# EUROPÄISCHE PATENTANMELDUNG

(11) **EP 4 000 567 A1**
(43) Veröffentlichungstag der Anmeldung: **25.05.2022**
(21) Anmeldenummer: 20206941.5
(22) Anmeldetag: 11.11.2020
(51) Int. Cl.: A61F 7/00, A61F 7/02

(54) **VORRICHTUNG ZUR WÄRMETHERAPEUTISCHEN BEHANDLUNG EINES AUSGEWÄHLTEN BEREICHS DES KÖRPERS EINES MENSCHEN ODER EINES TIERES UND VERFAHREN ZUM BETREIBEN DIESER VORRICHTUNG**

(71) Anmelder: Hilotherm Holding AG, 6317 Oberwil bei Zug (CH)
(72) Erfinder: Stegmann, Christian, 87545 Burgberg im Allgäu (DE)
(74) Vertreter: Wallinger, Michael

(57) **Zusammenfassung**

Die Erfindung betrifft eine Vorrichtung zur Verwendung bei der wärmetherapeutischen Behandlung eines ausgewählten Bereiches des Körpers eines Menschen oder eines Tieres, insbesondere zur Verwendung bei der Behandlung von Gewebeschädigungen nach operativen Eingriffen, von Verletzungen, von Entzündungen und von chronischen Erkrankungen des rheumatischen Formenkreises, und insbesondere zur Unterstützung von chemotherapeutischen Behandlungen, mit einer Temperiereinrichtung (1), die dazu vorgesehen ist, ein Temperierfluid zu temperieren, mit einer Wärmeübertragungseinrichtung (2), die zur Übertragung von Wärme auf den ausgewählten Bereich des Körpers eingerichtet ist, wobei die Temperiereinrichtung (1) mit der Wärmeübertragungseinrichtung, fluidisch verbunden ist, und ferner eingerichtet ist, die Wärmeübertragungseinrichtung (2), mit einem, die Wärmeübertragungseinrichtung (2), durchströmenden, wärmeübertragenden Temperierfluid, zu temperieren, und mit einer Steuereinrichtung (3), die mit der Temperiereinrichtung (1) und der Wärmeübertragungseinrichtung (2), verbunden ist, wobei die Steuereinrichtung (3) eingerichtet ist, die Temperiereinrichtung (1) anzusteuern, und um zu bewirken, dass die Wärmeübertragungseinrichtung (2), im Bereich einer Kontaktfläche zwischen der Wärmeübertragungseinrichtung (2), und dem zu temperierenden ausgewählten Bereich des Körpers zumindest teilweise eine in etwa gleichmäßige Temperatur aufweist. Die Erfindung betrifft ferner ein Verfahren zum Betreiben dieser Vorrichtung.

## Beschreibung

Die vorliegende Erfindung betrifft eine Vorrichtung zur Verwendung bei der wärmetherapeutischen Behandlung eines ausgewählten Bereiches des Körpers eines Menschen oder eines Tieres, und insbesondere zur Verwendung bei der Behandlung von Gewebeschädigungen nach operativen Eingriffen, von Verletzungen, von Entzündungen und von chronischen Erkrankungen des rheumatischen Formenkreises, und insbesondere zur Unterstützung von chemotherapeutischen Behandlungen sowie ein Verfahren zum Betreiben einer solchen Vorrichtung.

Unter dem Begriff "wärmetherapeutischen Behandlung" wird im Rahmen der vorliegenden Erfindung eine Behandlung verstanden, bei der dem menschlichen oder tierischen Körper Wärme zugeführt oder Wärme entzogen wird.

Dabei geht es vorzugsweise nicht um die Zufuhr oder Abfuhr von Wärme zum oder vom gesamten Körpers, wie dies zum Beispiel in einer Sauna und dem nachfolgenden Eintauchen in ein mit kaltem Wasser gefüllten Tauchbecken geschieht, sondern es geht vorzugsweise darum, einem ausgewählten Bereich des menschlichen Körpers, zum Beispiel den Gliedmaßen, Bereichen des Rückens, des Bauches, der Brust oder des Kopfes Wärme zuzuführen oder von dort abzuführen, um therapeutische Wirkungen zu erzielen.

Bekannte Vorrichtungen zur wärmetherapeutischen Behandlung weisen Temperiereinrichtungen auf, wie sie zum Beispiel in den europäischen Patenten EP 2 194 940 B1 und EP 2 462 906 B1 beschrieben werden. Diese Temperiereinrichtungen weisen ein Kühlaggregat und/oder ein Heizaggregat auf, mit dem ein Fluid, vorzugsweise Wasser, auf die gewünschte Temperatur gekühlt und/oder erwärmt wird. Vorzugsweise ist ferner eine oder mehrere Pumpen vorgesehen, mit denen das temperierte Fluid über Verbindungsschläuche zu einer Wärmeübertragungseinrichtung gefördert wird.

Die DE 20 2018 107 423 U1 zeigt ein Beispiel einer Wärmeübertragungseinrichtung, mit der die von der Temperiereinrichtung erzeugte Wärme auf den ausgewählten Bereich des Körpers übertragen wird. Die dort gezeigte Einrichtung, dort wie auch in der Praxis üblicherweise als Manschette bezeichnet, weist eine erste und eine zweite flexible Materiallage auf, zwischen denen Strömungsräume vorgesehen sind, die durch das temperierte Fluid durchströmt werden. Diese Manschetten können in vielen verschiedenen Formen für einen vorbestimmten Körperbereich gestaltet werden, zum Beispiel als Handmaschenmanschette, Unterarmmanschette, Kniemanschette, Wadenmanschette, Rundmanschette, Brustmanschette, Gesichtsmanschette. Sie können ferner als Trapezmanschetten oder Flächenmanschetten gestaltet werden, die je nach Größe und Formgebung dazu geeignet sind, auf einen beliebigen Bereich des Körpers aufgelegt zu werden. Zur Befestigung der Manschetten am Körper werden Verschlussmittel, Bänder und dergleichen verwendet.

Obwohl sich die vorgenannten Vorrichtungen in der Praxis bewährt haben, besteht der Wunsch nach einer weiteren Verbesserung. Der vorliegenden Erfindung liegt deshalb die Aufgabe zugrunde, die bekannten Einrichtung zu verbessern.

Diese Aufgabe wird erfindungsgemäß durch eine Vorrichtung nach Anspruch eins gelöst. Ein bevorzugtes Verfahren zum Betreiben dieser Vorrichtung ist Gegenstand des Anspruches 20. Zu bevorzugende Weiterbildungen der Erfindung sind Gegenstand der Unteransprüche.

Die Offenbarung der genannten Druckschriften EP 2 194 940 B17-07-27, EP 2 462 906 B17-07-27, DE 20 2018 107 423 U1 sowie die DE 10 2015 003 473 A1 werden im Wege der Referenz in die Offenbarung der vorliegenden Erfindung einbezogen.

Die erfindungsgemäße Vorrichtung weist eine Temperiereinrichtung auf, die dazu vorgesehen ist, ein Temperierfluid zu temperieren. Die Temperiereinrichtung ist hierfür mit einer Wärmeübertragungseinrichtung, die zur Übertragung von Wärme auf den ausgewählten Bereich des Körpers eingerichtet ist, fluidisch verbunden, und ferner eingerichtet, die Wärmeübertragungseinrichtung, mit einem, die Wärmeübertragungseinrichtung, durchströmenden, wärmeübertragenden Temperierfluid, zu temperieren. Die Vorrichtung weist ferner eine Steuereinrichtung auf, die mit der Temperiereinrichtung und der Wärmeübertragungseinrichtung, verbunden ist, wobei die Steuereinrichtung eingerichtet ist, die Temperiereinrichtung anzusteuern, um zu bewirken, dass die Wärmeübertragungseinrichtung, im Bereich einer Kontaktfläche zwischen der Wärmeübertragungseinrichtung, und dem zu temperierenden ausgewählten Bereich des Körpers zumindest teilweise eine in etwa gleichmäßige Temperatur aufweist.

Unter dem Begriff Steuereinrichtung ist eine Einrichtung zu verstehen, die aufgrund ihr zugeführten Daten oder Werte und/oder von einem Benutzer eingegebenen Daten oder Werte oder Einstellungen Teile der Vorrichtung entweder steuert und/oder regelt. Die Beeinflussung erfolgt vorzugsweise über Stelleinrichtungen. Unter einer Stelleinrichtung wird jedes elektrische oder mechanische Bauteil verstanden, welches geeignet ist, ein technisches System gerichtet zu beeinflussen.

Der ausgewählte Bereich des Körpers eines Menschen kann hierbei jede Region auf der Oberfläche des Körpers sein. Insbesondere kann der ausgewählte Bereich eines der Gliedmaßen, wie etwa ein Arm oder ein Bein, vorzugsweise eine Hand oder ein Fuß, betreffen. Ferner kann der ausgewählte Bereich auch einen Bereich der Brust, des Bauches des Rückens oder des Kopfes betreffen. Die Haut des Patienten im ausgewählten Bereich wird nachfolgend auch als Hautkontaktfläche bezeichnet.

Unter einer Fluidverbindung, oder fluidisch verbundenen, Verbindung wird eine Verbindung verstanden, die den Austausch eines flüssigen oder gasförmigen Fluids von einem Ort zu einem anderen Ort ermöglicht. Typische Fluidverbindungen erfolgen vorzugsweise über einen Schlauch oder ein Rohr. Die Fluidverbindungen sind vorzugsweise teilweise oder vollständig thermisch isoliert, um den Wärme- bzw. Kälteverlust während der Leitung des Fluids möglichst gering zu halten, um einerseits einen energiesparenden Aufbau, und andererseits ein möglichst genaues Einhalten der Zieltemperatur, die die Wärmeübertragungseinrichtung im Bereich des Hautkontakts erreichen soll, zu gewährleisten.

Gemäß der Erfindung wird ein wärmeübertragendes Temperierfluid verwendet. Unter einem wärmeübertragenden Temperierfluid ist ein Fluid zu verstehen, welches funktional eingerichtet ist, die ihm innwohnende Wärme, in Form von Wärmeenergie, zu einem Zielort zu transportieren. Vorteilhafterweise ist ein wärmeübertragendes Temperierfluid daher eine Flüssigkeit, welche geeignet ist, eine große Menge an Wärmeenergie zu speichern oder aufzunehmen. Vorzugsweise wird als Temperierfluid Wasser verwendet, es ist aber auch bevorzugt eine Wasser-Salzmischung oder ein geeignetes mineralisches oder synthetisches Öl zu verwenden.

Der Begriff "verbunden" kann im Zusammenhang der Erfindung "direkt verbunden", wie vorzugsweise ein Kabel, ein Schlauch, oder "indirekt verbunden", wie vorzugsweise über Licht, Infrarot, oder Funk nach dem Wlan- oder Bluetooth-Standard bedeuten. Die Temperiereinrichtung ist hierbei mit der Wärmeübertragungseinrichtung direkt, d.h. mittels wenigstens einer Fluidleitung verbunden, während die Temperiereinrichtung mit der Steuereinrichtung auch indirekt, also ohne Leitung über Funk oder dergleichen verbunden sein kann.

Als den Bereich einer Kontaktfläche zwischen der Wärmeübertragungseinrichtung und dem zu temperierenden Bereich des Körpers wird derjenige Bereich bezeichnet, an dem eine Kontaktfläche der Wärmeübertragungseinrichtung einen geringen Abstand zu der Hautoberfläche des zu temperierenden Bereichs des Körpers aufweist und bevorzugt unmittelbar auf der Hautoberfläche aufliegt.

Die Wärmeübertragungseinrichtung soll gemäß der Erfindung eine in etwa gleichmäßige Temperatur auf dem zu temperierenden, ausgewählten Bereich des Körpers erzeugen. Unter einer in etwa gleichmäßigen Temperatur ist hierbei zu verstehen, dass alle Punkte in diesem Bereich der Kontaktfläche der Wärmeübertragungseinrichtung in einem Intervall von 10° C, bevorzugt 5°C, besonders bevorzugt 3° C, ferner besonders bevorzugt 1°C liegen.

In einer bevorzugten Ausführungsform weist die Wärmeübertragungseinrichtung hierfür wenigstens eine Wärmeübertragungseinheit auf, welche dafür vorgesehen ist, in Kontakt mit dem ausgewählten Bereich des Körpers gebracht zu werden. Die Wärmeübertragungseinheit besteht dabei aus einem im wesentlichen flexiblen Material, in welchem eine oder mehrere, von dem Temperierfluid durchströmbare, Temperierfluidkammern angeordnet sind.

Der zu temperierende Bereich des Körpers ist in der Regel nicht eben, sondern weist konkave und/oder konvexe Bereiche auf. Im Rahmen der vorliegenden Erfindung wurde festgestellt, dass diese Unebenheiten dazu führen können, dass sich zwischen der Wärmeübertragungseinheit und der Hautfläche des zu behandelnden Körpers einzelne Luftpolster bilden, die den betroffenen örtlichen Bereich der Hautpartien des ausgewählten Bereichs thermisch von der Wärmeübertragungseinheit isolieren. Dadurch wird die Temperierung des ausgewählten Bereiches ungleichmäßig und die erwünschten Folgen der Temperierung insbesondere eine Erhöhung oder eine Erniedrigung der Durchblutung, erfolgt nicht gleichmäßig.

Um diesen Effekt der örtlich begrenzten thermischen Isolation zu vermindern weist, die Wärmeübertragungseinrichtung ein im wesentlich flexibles Material auf, um den Abstand zwischen Wärmeübertragungseinheit und dem zu temperierenden Bereich über die ganze dreidimensionale Fläche möglichst klein zu halten, wobei die Flexibilität vorzugsweise ein direktes Aufliegen über den zu temperierenden ausgewählten Bereich ermöglicht.

Die Temperierfluidkammern sind dabei derart innerhalb der Wärmeübertragungseinheit angeordnet, dass bei einem Durchströmen der Temperierfluidkammern die Wärme des Temperierfluids möglichst gleichmäßig auf den zu temperierenden Bereich des Körpers abgegeben wird.

Um die Wärme dabei möglichst gleichmäßig abzugeben weist das im wesentlich flexible Material der Wärmeübertragungseinheit in einer bevorzugten Ausführungsform dabei wenigstens eine erste flexible Materiallage mit wenigstens einer ersten Hautkontaktfläche, welche zumindest teilweise einem ausgewählten Bereich des Körpers eines Menschen oder eines Tieres zugewandt ist, und wenigstens eine, von der ersten Hautkontaktfläche abgewandten, erste Temperierfluidkontaktfläche auf. Zusätzlich weist die Wärmeübertragungseinheit wenigstens eine zweite flexible Materiallage mit wenigstens einer zweiten Temperierfluidkontaktfläche, die zwischen sich und der ersten Temperierfluidkontaktfläche der ersten flexiblen Materiallage zumindest teilweise die eine, oder mehrere, von dem wärmeübertragenden Temperierfluid durchströmbare Temperierfluidkammer begrenzt.

Die Wärmeübertragungseinheit weist dabei ferner wenigstens eine erste Zuleitung, um der Wärmeübertragungseinheit ein wärmeübertragendes Temperierfluid zuzuführen, und wenigstens eine zweite Zuleitung, um ein wärmeübertragendes Temperierfluid von der Wärmeübertragungseinheit abzuführen, auf. Die erste und die zweite Zuleitung sind dabei mit der Temperiereinrichtung verbunden, um das Temperierfluid in einem Kreislauf auf die gewünschte Temperatur zu erwärmen oder abzukühlen.

Durch die Verwendung flexibler Materialien weist die Wärmeübertragungseinheit dabei wenigstens eine Form auf, oder kann eine solche Form annehmen, in welcher sie den ausgewählten Bereich des Körpers eines Menschen oder eines Tieres abdeckt und/oder zumindest teilweise umschließt.

In einer bevorzugten Ausführungsform weist die Wärmübertragungseinrichtung, zur Verminderung des vorstehend beschriebenen Effekts der örtlichen thermischen Isolation durch Luftpolster ferner eine Druckeinrichtung auf, um einen Anliegedruck der Wärmeübertragungseinheit auf den ausgewählten Bereich des Körpers zu erzeugen bzw. zu verstärken um dadurch das Auftreten von thermisch isolierenden Luftpolster zwischen der Wärmeübertragungseinheit und dem zu temperierenden Bereich wenigstens teilweise oder aber weitgehend vollständig zu verringern. Die Druckeinrichtung weist dabei wenigstens eine Druckeinheit und eine Abstützeinrichtung auf, wobei die Druckeinrichtung durch die Abstützeinrichtung an einer Bewegung in einer Richtung entgegen der Hautkontaktfläche des ausgewählten Bereichs gehindert ist.

Die Druckeinheit ist dazu vorgesehen, die Wärmeübertragungseinheit wenigstens teilweise mit einem Druck zu beaufschlagen, welcher dann als Anliegedruck der Wärmeübertragungseinrichtung vom zu behandelnden Teil des Körpers aufgenommen wird. Die Druckeinheit weist hierfür wenigstens eine Druckkammer mit einem vorbestimmten oder einem veränderbaren Innendruck auf.

Die Verwendung einer mit einem Fluid gefüllten Druckkammer hat besondere Vorteile. In einer mit einem Fluid gefüllten Druckkammer ist der Druck innerhalb des Fluid immer gleich groß. Dies bedeutet, dass die Wärmeübertragungseinheit mit gleichmäßigem Druck auf die Hautpartien des ausgewählten Bereiches gedrückt werden. Dadurch kann sich das flexible Material der Wärmeübertragungseinheit besonders gut an konvexe und/oder konkave Bereiche des ausgewählten Bereichs anpassen, wodurch das Auftreten von Luftpolstern vermieden und eine besonders gute Wärmeübertragung erzielt wird.

Der Anliegedruck stellt dabei denjenigen Anteil der Flächenkraft dar, der von der Druckeinheit über die Wärmeübertragungseinheit auf den zu temperierenden, ausgewählten Bereich des Körpers weitergeleitet wird.

Um dem Anliegedruck aufzubauen ist eine Abstützeinrichtung vorgesehen. Unter einer Abstützeinrichtung wird jede Art Einrichtung verstanden, die geeignet ist, Flächenkräfte aufzunehmen, die dem Anliegedruck entgegengerichtet sind.

In einer bevorzugten Ausführungsform weist die wenigstens eine Druckeinheit eine mit einem Druckfluid gefüllte Kammer auf, wobei die wenigstens eine mit einem Druckfluid gefüllte Kammer durch ein flexibles Material umgrenzt ist. Unter einem Druckfluid wird ein Fluid verstanden, dass kompressibel, vorzugsweise Luft oder ein Gas oder inkompressibel, vorzugsweise Wasser, eine Wasser-Salzmischung, eine Wasser-Ölmischung, oder ein Öl sein kann. Es sind aber auch andere Fluide zur Verwendung als Druckfluid möglich.

In einer bevorzugten Ausführungsform ist die wenigstens eine, mit einem Druckfluid gefüllte Kammer mit einer Druckleitung verbunden. Das Druckfluid in der wenigstens einen mit einem Druckfluid gefüllten Kammer ist dabei entweder manuell über Muskelkraft durch einen Benutzer, und/oder über einen Verdichter, über die Druckleitung, die mit der wenigstens einen mit einem Druckfluid gefüllten Kammer verbunden ist, mit Druck beaufschlagbar. Dadurch ist ein Innendruck in der wenigstens einen mit einem Druckfluid gefüllten Druckkammer veränderbar. Durch den veränderbaren Innendruck ist somit auch der Anliegedruck der Wärmeübertragungseinrichtung auf den Teil des Körpers einstellbar. Vorzugsweise ist eine Einrichtung vorgesehen, die über die Muskelkraft eines Menschen betätigt wird.

Unter einem Verdichter wird dabei eine Maschine verstanden, die dem Zweck dient, dem Druckfluid mechanische Arbeit zuzuführen und dadurch den Innendruck zu erhöhen. Handelt es sich bei dem Fluid um eine Flüssigkeit stellt der Verdichter eine Pumpe dar.

In einer Ausführungsform weist die Druckeinrichtung ein Ablassventil zum Absenken des Innendrucks der Druckeinheit auf.

In einer weiteren bevorzugten Ausführungsform ist die wenigstens eine Druckeinheit nicht aus einer mit einem Druckfluid gefüllten Druckkammer gebildet, sondern aus einem elastischen Material hergestellt, und ebenfalls zwischen der Abstützeinrichtung und der Wärmeübertragungseinheit angeordnet. Das elastische Material kann hierbei ein Schaumstoff, ein Gummi, oder eine Kombination dieser oder ähnlicher Materialien sein. Die Elastizität kann dabei über das Volumen der Druckeinheit konstant sein, es sind jedoch auch Ausführungsformen denkbar in denen die Elastizität der Druckeinheit über das Volumen variiert um mögliche Druckspitzen zu vermeiden, und/oder gezielt einen erhöhten Druck, vorzugsweise über kleine Radien des zu behandelnden Teils des Körpers, anzulegen.

In einer bevorzugten Ausführungsform ist die Abstützeinrichtung der Druckeinrichtung aus einem wenig flexiblen Material hergestellt.

Unter einem wenig flexiblen Material ist hierbei ein Material mit einem höheren Elastizitätsmodul wie vorzugsweise Hartplastik zu verstehen. Wesentlich ist hierbei vor allem, dass das für die Abstützeinrichtung verwendete Material eine höhere Steifigkeit als die Druckeinheit aufweist, um den Innendruck der Druckeinheit auf die Kontaktfläche zwischen der Wärmeübertragungseinheit und dem ausgewählten Bereich des Körpers und der Wärmeübertragungseinheit zu leiten.

In einer bevorzugten Ausführungsform weist die Abstützeinrichtung der Druckeinrichtung um den ausgewählten Bereich des Körpers eines Menschen oder Tieres dabei ferner eine Verbindungseinrichtung mit wenigstens einem Fixierelement auf. Die Verbindungseinrichtung ist dabei derart ausgebildet, dass sie die Druckeinrichtung zumindest teilweise umgreift und in dieser umgreifenden Stellung Stützkräfte auf die Druckeinrichtung ausübt, welche zumindest teilweise dazu beitragen, die Druckeinrichtung in einem Benutzungszustand an dem ausgewählten Bereich des Körpers eines Menschen oder eines Tieres zu fixieren. Die Verbindungseinrichtung mit wenigstens einem Fixierelement kann dabei stufenlos verstellbar sein, wie vorzugsweise durch einen Gurt, oder dedizierte Fixierpunkte beinhalten, wie vorzugsweise Druckknöpfe, oder aus einer Kombination aus beidem bestehen.

Unter einem Benutzungszustand wird derjenige Zustand verstanden, in dem die Wärmeübertragungseinrichtung an dem ausgewählten Bereich des Körpers anliegt.

In einer bevorzugten Ausführungsform ist ferner wenigstens eine Sensoreinrichtung vorgesehen, die mit der Steuereinrichtung verbunden ist. Die Sensoreinrichtung ist vorzugsweise dafür eingerichtet, an bestimmten Positionen der Vorrichtung ein Sensorsignal zu messen. Vorzugsweise wird mit der Sensoreinrichtung die Temperatur und/oder der Anliegedruck zwischen dem ausgewählten Bereich des Körpers und der Hautkontaktfläche und/oder in der Wärmeübertragungseinrichtung und/oder in einem dem Körper zugewandten Randbereich der Wärmeübertragungseinrichtung gemessen.

Unter einer Sensoreinrichtung ist jede Einrichtung zu verstehen die einen Sensor zur Messung eines physikalischen Zustands aufweist, und eingerichtet ist, diesen Zustand in ein Sensorsignal umzuwandeln. Das Sensorsignal kann dabei optisch, akustisch oder digital vorliegen.

In einer bevorzugten Ausführungsform ist die wenigstens eine Sensoreinrichtung eine Mehrzahl von Sensoreinrichtungen, die vorzugsweise zumindest teilweise in einem Raster, vorzugsweise im Randbereich, oder auf der in der Gebrauchsstellung der Haut zugewandten Fläche der ersten Materiallage, angebracht sind. Das Raster kann dabei ein Raster mit identischen Abständen, oder ein Raster mit nahezu identischen Abständen oder ein Raster mit unterschiedlichen Abständen, oder eine Kombination hiervon sein.

In einer bevorzugten Ausführungsform ist das gemessene Sensorsignal dabei ausgewählt aus der Gruppe Druck, Stromstärke, Temperatur. Es sind aber auch andere physikalische Größen denkbar.

In einer bevorzugten Ausführungsform ist wenigstens eine Anzeigeeinrichtung vorgesehen. Die Anzeigeeinrichtung ist mit der Steuereinrichtung verbunden und eingerichtet, das von der Sensoreinrichtung gemessene Sensorsignal über die Anzeigeeinrichtung an einen Benutzer auszugeben.

Die Anzeigeeinrichtung kann dabei jede Form der Darstellungsmöglichkeit umfassen, die es ermöglicht einem Benutzer Auskunft über den Zustand der einzelnen Einrichtungen und/oder das gemessene Sensorsignal zu geben. So kann die Anzeige optisch, vorzugsweise in Form eines Bildschirms, akustisch, vorzugsweise in Form eines hörbaren Tons, aber auch haptisch, vorzugsweise in Form einer Vibration, erfolgen. Es sind aber auch andere Formen der Anzeige denkbar.

Um dem wärmeübertragenden Fluid die zur Temperierung des ausgewählten Bereichs des Körpers die notwendige Energie zu- oder abzuführen, ist eine Temperiereinrichtung vorgesehen. Die Temperiereinrichtung weist vorzugsweise ein Gehäuse, vorzugsweise eine Temperierfluidversorgungseinrichtung, durch welche ein wärmeübertragendes Temperierfluid in dem Gehäuse bereitgestellt wird, und wenigstens eine vorzugsweise im Gehäuse angeordneten Temperiereinheit, durch die das wärmeübertragende Temperierfluid temperiert, auf. Ferner weist die Temperiereinrichtung wenigstens eine Temperierfluidabströmungseinrichtung, durch welche das wärmeübertragende Temperierfluid mit einer im Wesentlichen vorbestimmten Temperatur aus dem Gehäuse herausgeführt wird, und vorzugsweise wenigstens eine vorzugsweise im Gehäuse angeordnete Pumpeneinrichtung, welche das durch das Gehäuse strömende wärmeübertragende Temperierfluid mit Druck beaufschlagt, auf.

Die Temperiereinheit weist vorzugsweise ein elektrisches Heizaggregat auf. Weiter vorzugsweise weist die Temperiereinheit wenigstens eine konventionelle Kühleinheit, in der ein Kältemittel komprimiert, über ein Expansionsventil entspannt und über einen Kondensator kondensiert wird.

Besonders bevorzugt weist die Temperiereinheit, zusätzlich oder ausschließlich, wenigstens ein Temperierelement auf, welches als thermoelektrisches Element, insbesondere als Peltier-Element ausgebildet ist. Vorzugsweise ist ferner ein von einem Temperierfluid durchströmbares Wärmetauscherelement zur Übertragung von Wärme auf das oder von dem Temperierfluid, und vorzugsweise wenigstens eine Kühleinheit vorgesehen.

Vorzugsweise verwendet die Vorrichtung wenigstens eine von den drei nachfolgend beschriebenen Gruppen von Sensoren.

Eine erste Gruppe von Sensoren ist dafür vorgesehen, den Zustand der Wärmeübertragungseinrichtung und gegebenenfalls auch der Zuleitungen zu erfassen.

Dazu gehören Temperatursensoren, die die Temperatur an vorbestimmten Stellen der Wärmeübertragungseinrichtung und der Zuleitungen erfassen, Drucksensoren, die die Druckkräfte der Druckeinheit innerhalb der Wärmeübertragungseinrichtung messen und Sensoren zur Strömungsmessung, mit denen die Strömungsgeschwindigkeit und oder die Menge des zu- und/oder abgeführten Temperierfluid erfasst werden kann.

Eine zweite Gruppe von Sensoren ist innerhalb der Temperiereinrichtung vorgesehen. Auch hier werden vorzugsweise Temperaturen, Druck und Strömungsgeschwindigkeit bzw. Strömungsvolumen erfasst.

Eine dritte Gruppe von Sensoren ist dafür vorgesehen, Messungen von physiologischen Daten am Patienten vorzunehmen, die vorzugsweise von der Steuereinrichtung verwendet werden, um den Betrieb der Vorrichtung und insbesondere die Temperatur der Wärmeübertragungseinrichtung zu steuern und/oder zu regeln.

Vorzugsweise werden Temperatursensoren verwendet, die vorzugsweise als dünne Elektroden im ausgewählten Bereich des Körpers angeordnet werden, der durch die Vorrichtung temperiert werden soll. Ferner werden vorzugsweise Drucksensoren verwendet, ebenfalls vorzugsweise als dünne Elektroden, mit denen der Anliegedruck der Wärmeübertragungseinrichtung erfasst werden kann.

Weiter vorzugsweise werden Sensoren verwendet, die den physiologischen Zustand des behandelten Patienten erfassen. Dazu werden ein oder mehrere Sensoren aus einer Untergruppe ausgewählt, die nachfolgend genannten Sensoren umfasst:, Sensoren, die die Herzfrequenz messen, Sensoren, die ein EKG aufnehmen, Sensoren, die den Blutdruck messen, und vorzugsweise Sensoren, die die Durchblutung messen.

In einer bevorzugten Ausführungsform ist die Steuereinrichtung eingerichtet, aufgrund der von den Sensoreinrichtungen bzw. Sensoren gemessenen Signal(e) einen oder mehrere Regelkreise derart zu bilden, dass ein Bereich einer Kontaktfläche zwischen der Wärmeübertragungseinrichtung, und dem zu temperierenden Teils des Körpers zumindest in einem Bereich eine in etwa gleichmäßige, vorgewählte Temperatur aufweist.

Die Vorrichtung weist vorzugsweise eine oder mehrere Stell- oder Regeleinrichtungen auf, die es erlauben, Parameter der Vorrichtung wie die Temperatur des Fluids, die Durchflussmenge, die geförderte Fluidmenge und dergleichen zu verändern. Diese Stell- oder Regeleinrichtungen können elektrische Regeleinrichtungen sein, die vorzugsweise eine Spannung, eine Frequenz und oder einen Strom beeinflussen. Weiter bevorzugt können die Regeleinrichtungen mechanische Regeleinrichtungen sein, die vorzugsweise den Durchflusswiderstand verändern und/oder den Durchfluss stoppen können oder sie können elektromechanisch ausgestaltet sein.

Die Steuereinrichtung ist mit wenigstens einem, vorzugsweise aber mit zwei oder weiter vorzugsweise mit einer Vielzahl von Bedienelementen ausgestattet, die es ermöglichen, die gewünschten Behandlungsparameter einzustellen. Vorzugsweise gehört dazu die Vorgabe von Betriebs- und Pausenzeiten sowie die Vorgabe von Temperaturen oder Temperaturintervallen.

Wenn die Vorrichtung zur Kühlung eingesetzt wird, sind, je nach Anwendungsbereich, folgende Temperaturbereiche besonders vorteilhaft, die von der jeweiligen Vorrichtung abgedeckt werden sollten. Für den Einsatz im Krankenhaus etc. empfiehlt sich ein Temperaturbereich von 10-35 °C, für Krankenhaus und ambulante Behandlungen ein Temperaturbereich von 12-22 °C und für die Behandlung im Privatbereich ein Temperaturbereich von 15-22 °C. Es ist aber auch bevorzugt, die Vorrichtung so auszugestalten, dass sie einen größeren Temperaturbereich von 10-35 °C abdeckt, oder aber auch einen Temperaturbereich von 10-43 °C.

Vorzugsweise weist die Steuereinrichtung eine Prozessoreinrichtung und eine Speichereinrichtung auf. In der Speichereinrichtung sind vorzugsweise ein oder mehrere Programmroutinen abgespeichert, die einzeln oder zusammen dazu dienen, mittels der Prozessoreinrichtung den Betrieb der Steuereinrichtung und vorzugsweise auch Teilen der Vorrichtung zu beeinflussen bzw. zu steuern. Die Programmroutinen sind vorzugsweise vorgegeben und fest im der Speichereinrichtung gespeichert sein. Weiter vorzugsweise können die Programmroutinen vom Benutzer verändert und/oder zusätzliche Programmroutinen in die Speichereinrichtung eingegeben werden.

Insbesondere, aber nicht ausschließlich, bei einer Vorrichtung, die nur oder auch im privaten Bereich außerhalb eines Krankenhauses oder einer sonstigen medizinischen Einrichtung Verwendung findet, kann ein Teil oder die gesamte Steuerung der Steuereinrichtung durch eine externe Prozessoreinrichtung erfolgen. Vorzugsweise wird dafür ein stationärer oder transportabler Computer, wie vorzugsweise ein Laptop verwendet. Weiter vorzugsweise wird als externe Prozessoreinrichtung eine mobile Kommunikationseinrichtung, insbesondere ein Mobiltelefon oder besonders vorzugsweise ein Smartphone verwendet. Vorzugsweise wird die mobile Kommunikationseinrichtung, insbesondere ein Smartphone, mit externen Sensoren verbunden, die physiologische Daten des Patienten erfassen können. Vorzugsweise werden dafür Sensoren verwendet, die am Körper des Patienten befestigt werden können, wie zum Beispiel Armbänder mit Sensoren oder eine Smartwatch. Die auf der mobilen Kommunikationseinrichtung verwendeten Programme zum Erfassen physiologischer Daten wie vorzugsweise die Herzfrequenz und EKG Funktionen können dann zur Steuerung der Vorrichtung verwendet werden.

Vorzugsweise weist die Vorrichtung einen Alarmmodus auf. Dieser Alarmmodus wird vorzugsweise dann ausgelöst, wenn eine Temperatur in einem nicht erwünschten Bereich liegt. Dies kann vorzugsweise der Fall sein, wenn die Fluidtemperatur über einen vorbestimmten Wert ansteigt oder wenn, weiter vorzugsweise, eine Gehäusetemperatur einen unerwünschten Wert annimmt. Ferner kann der Alarmmodus auch ausgelöst werden, wenn die physiologischen Werte, die die mit dem Patienten verbundenen Sensoren erfassen, in einem nicht erwünschten Bereich liegen.

Bei den vorerwähnten Beispielen gemäß der Erfindung ist ein Verfahren zum Betreiben einer Vorrichtung zur wärmetherapeutischen Behandlung eines ausgewählten Bereiches des Körpers eines Menschen oder eines Tieres, vorgesehen, wobei das Verfahren folgende Schritte aufweist. Zu Beginn wird eine Wärmeübertragungseinrichtung mit einer Druckeinrichtung an den ausgewählten Bereich des Körpers in einen Benutzungszustand in Kontakt gebracht. Über die Druckeinrichtung wird anschließend ein auf den ausgewählten Bereich des Körpers wirkender Anliegedrucks angelegt. Über eine mit der Wärmeübertragungseinrichtung verbundene Temperiereinrichtung wird der ausgewählten Bereich des Körpers temperiert, indem eine Wärmeübertragungseinheit der Wärmeübertragungseinrichtung von einem wärmeübertragenden Temperierfluid durchströmt wird, so dass im Bereich einer Kontaktfläche zwischen der Wärmeübertragungseinrichtung, und dem zu temperierenden Teils des Körpers zumindest in einem Bereich eine in etwa gleichmäßige Temperatur aufweist.

Unter einem in Kontakt bringen der Wärmeübertragungseinrichtung kann vorzugsweise das Anlegen einer, eine Wärmeübertragungseinrichtung enthaltende, Manschette um eine Hand eines Patienten verstanden werden. Ferner kann unter in Kontakt bringen auch das Auflegen eines Körperteils auf eine auf einem Kissen ruhende Wärmeübertragungseinrichtung verstanden werden.

In einer bevorzugten Ausführungsform weist das Verfahren ferner folgenden Schritt auf:
Ein Innendruck einer Druckeinheit der Druckeinrichtung wird eingestellt, wobei die Druckeinheit wenigstens eine mit einem Druckfluid gefüllte Kammer aufweist. Der Innendruck der wenigstens einen mit einem Druckfluid gefüllten Kammer ist dabei manuell über Muskelkraft eines Benutzers und/oder über eine externe Druckquelle einstellbar.

In einer bevorzugten Ausführungsform weist das Verfahren ferner folgende Schritte auf:
Über eine mit der Wärmeübertragungseinrichtung verbundene Sensoreinrichtung wird ein Sensorsignal gemessen.
Über eine Steuereinrichtung wird daraufhin, basierend auf dem gemessenen Sensorsignal, eine mit der Steuereinrichtung verbundene Druckeinrichtung gesteuert. Dieses Steuern kann auch als Regelkreis bezeichnet werden.

Nachfolgend werden therapeutische Behandlungen beschrieben, für die sich die erfindungsgemäße Vorrichtung besonders eignet:
Gezielte Wärmebehandlungen vor einem chirurgischen Eingriff verbessern die kapillare Durchblutung und fördern die Heilung nach dem Eingriff. Dazu wird mit der erfindungsgemäßen Vorrichtung ca. 18 Stunden vor dem Eingriff mehrmals Wärme aufgebracht, wobei eine Temperierung des Gewebes auf 41-43°C besonders vorteilhaft ist. Diese Temperierung wird zum Beispiel dreimal für jeweils eine halbe Stunde aufgebracht, unterbrochen durch eine jeweils ebenfalls eine halbe Stunde dauerten Kühlphase bei Raumtemperatur.

Dazu wird in der Vorrichtung ein Ablaufprogramm gespeichert oder eingegeben, das den Zeitablauf und die verschiedenen Bestandteile der Vorrichtung steuert. Besonders bevorzugt werden dafür physikalische (Temperatur, Druck etc.) oder physiologische Daten verwendet, die mit den Sensoren gemessen werden

Nach einem chirurgischen Eingriff, insbesondere aber auch nach Verletzungen und bei chronischen Erkrankungen und daraus resultierenden Schwellungen, Einblutungen, Hämatomen, Ödemen und Entzündungen fördert die Kühlung des betroffenen Gewebes mit der erfindungsgemäßen Vorrichtung die Heilung und vermindert die Schmerzintensität, was eine deutlich reduzierte Arzneimittelgabe zur Folge haben kann. Diese therapeutische Wirkung lässt sich dadurch erklären, dass das betroffene Gewebe auf den Eingriff bzw. die Verletzung mit einer Entzündung und dadurch einer Übererwärmung reagiert, wodurch sich der Sauerstoffbedarf erhöht. Die Kühlung des Gewebes reduziert diesen Sauerstoffbedarf und verhindert das Absterben von Zellen. Dazu wird die Vorrichtung so gesteuert, dass eine bestimmte Zieltemperatur erreicht wird. Die Zieltemperatur zur Erzielung des Gewebes kann zum Beispiel 14,5 °C sein. Das Erreichen der Zieltemperatur kann durch die Sensoren überwacht werden und ferner kann der physiogische Zustand des Patienten überwacht werden, und die Steuereinrichtung gegebenenfalls abgeschaltet werden, falls sie Werte für die Herzfrequenz, die Durchblutung oder dergleichen in einem unerwünschten Bereich liegen.

Eine besondere Bedeutung haben wärmetherapeutische Maßnahmen auch bei der Behandlung von Krebs mittels der Chemotherapie. Das zur Reduzierung der Krebszellen verwendete Chemotherapeutikum dringt in die Blutkapillaren von Händen und Füßen und kann zur gefürchteten Chemotherapie-induzierten Polyneuropathie (CIPN) oder Hand-Fuß-Syndrom (HFS) mit dauerhafter Schädigung des Gewebes und insbesondere der Nervenzellen führen. Diese äußert sich in leichten Fällen als Gefühlsstörungen, "Kribbeln" und dergleichen, in schweren Fällen zu einem weitgehenden oder vollständigen Taubheitsgefühl, den Gebrauch der Hände aber auch das Gehen erschweren kann. Ferner wird auch über Schmerzen mit zum Teil hoher Intensität berichtet. In schweren Fällen kommt es auch häufig zur Onycholyse, d.h. der Ablösung eines oder mehrerer Nägel.

Der bevorzugte Befall des sensiblen Nervensystems ist nach dem gängigen Verständnis durch die Blutversorgung dieser Nervenzellen mit gefensterten Kapillaren begründet. Das heißt, die Eiweißzusammensetzung der Zellwand ist anders, also durchlässiger für bestimmte Chemotherapeutika. Somit entfalten sie elementare Schäden an den Nervenzellen. Die Schädigung der zellulären wie auch der mitochondrialen DNA, ein gestörter Kalziumhaushalt und oxidativer Stress fördern die Auflösung der Ganglienzellen. Da die längsten Nervenfasern empfindlicher gegenüber diesen schädlichen Einflüssen sind, präsentiert sich die CIPN meist als längenabhängig mit Betonung der unteren Extremitäten.

Die Kühlung von Händen und Füßen oder anderen Bereichen des Körpers bei der Chemotherapie mit der erfindungsgemäßen Vorrichtung führt dazu, dass die Durchblutung in diesen Bereichen vermindert wird, wodurch weniger Therapeutikum die Blutkapillaren erreicht und das Entstehen der Polyneuropathie verhindert werden kann. Auch dies kann mit der erfindungsgemäßen Vorrichtung und mit dem erfindungsgemäßen Verfahren zum Betreiben der Vorrichtung vorteilhaft erreicht werden.

Die Kühlung wird vorzugsweise in einem Temperaturbereich zwischen 8° und 30° C, besonders bevorzugt aber in einem Bereich zwischen 10 °C und 14 °C durchgeführt. Ganz besonders bevorzugt ist eine Kühltemperatur im Bereich von 10-12°C.

Vorzugsweise wird die Wärmebehandlung, die vorstehend in Bezug auf chirurgische Eingriffe erläutert wurde, auch bei der Behandlung im Zusammenhang mit der Chemotherapie in Form einer Kältebehandlung angewendet. Dazu wird mit der erfindungsgemäßen Vorrichtung vorzugsweise ca. 30 min vor der Gabe des Chemotherapeutikums Kälte aufgebracht, wobei eine Temperierung der Gewebe- oder Hautoberfläche des ausgewählten Bereichs auf 10 - 12 C° besonders vorteilhaft ist. Diese Temperierung wird vorzugsweise während der gesamten Verabreichung des Chemotherapeutikums aufgebracht sowie vorzugsweise 1 Std. bis 3 Std. über den Abschluß der Verabreichung des Chemotherapeutikums hinaus.

Vorzugsweise werden als Chemotherapeutikum für die Chemotherapie zytostatische Arzneistoffe, wie z.B. Carboplatin, Capecitabin, 5-Fu, Cyclophosphamid, Cytarabin. Docetaxel, Doxorubicin, Oxaliplatin, Paclitaxel, Sorafinid und Sunitinib verwendet. Diese Bezeichnungen sind Handelsnamen, die für die jeweiligen Herstellungs- bzw. Vertriebsfirmen geschützt sind.

Um für jeden Patienten die für ihn optimale Kühltemperatur einstellen zu können, kann die Temperiereinrichtung das Temperierfluid mit einer Genauigkeit von 1 °C, bevorzugt aber mit einer Genauigkeit zwischen 0,5 und 0,2 °C einstellen. Für den gewünschten therapeutischen Effekt kann es dabei wesentlich sein, dass die gewünschte Temperatur, in der vorliegenden Beschreibung auch als Zieltemperatur bezeichnet, auch während des Kühlvorganges möglichst genau gehalten wird.

Bei der dieser Anwendung in Verbindung mit einer Chemotherapie ist die Vorrichtung besonders vorteilhaft mit einer Druckeinrichtung ausgestattet. Da es darum geht, die Durchblutung der Blutkapillaren herabzusetzen, ist es hier von besonderem Vorteil, wenn die Wärmeübertragungseinheit in gleichmäßigem Kontakt, d.h. möglichst ohne thermisch isolierende Luftpolster mit den Hautpartien des ausgewählten Bereiches des Körpers des Patienten steht. Dies wird durch die Druckeinrichtung erreicht.

Durch eine Zeitsteuerung der Vorrichtung kann zum Beispiel die Zeit vorgegeben werden, die erforderlich ist, um die Zieltemperatur zu erreichen, bevor mit der Zufuhr des Chemotherapeutikums begonnen wird.

Stattdessen können aber auch Temperatursensoren an der Wärmeübertragungseinrichtung verwendet werden, um das Erreichen der Zieltemperatur zu erfassen und um dann ein entsprechendes Signal auszugeben.

Schließlich kann hier in besonders vorteilhafter Weise auch ein Sensor für die Messung der Durchblutung Verwendung finden. Der Kühlprozess wird dann begonnen und fortgesetzt, bis die Durchblutung auf einen vorbestimmten Wert abgesunken ist. Durch die Überwachung der physiologischen Werte des Patienten durch die Vorrichtung, wie die Herzfrequenz und den Blutdruck, kann gleichzeitig sichergestellt werden, dass die Kühlung nicht zu einem unerwünschten Zustand des Patienten führt.

Bei den vorerwähnten Beispielen wird davon ausgegangen, dass die Temperiereinrichtung den ausgewählten Bereich auf eine bestimmte Zieltemperatur erwärmt oder abkühlt. Statt einer konstanten Zieltemperatur kann die Temperatur aber auch während der Behandlung verändert werden. Dies kann durch eine Modulation der Temperatur/Zeitkurve erfolgen. Diese Modulation kann vorzugsweise in der Temperatur/Zeitkurve einen rechteckförmigen, sinusförmigen, sägezahnförmigen oder trapezförmigen Verlauf aufweisen.

Weitere vorteilhafte Ausgestaltungen der Erfindung werden in der nachfolgenden Beschreibung in Zusammenhang mit den beiliegenden Zeichnungen erläutert. Dabei zeigen:
**Fig. 1****:** eine erfindungsgemäße Vorrichtung zur Verwendung bei der wärmetherapeutischen Behandlung;
**Fig. 2****:** eine erfindungsgemäße Wärmeübertragungseinheit in Handschuhform im aufgeklappten Zustand;
**Fig. 3****:** eine erfindungsgemäße Druckeinrichtung im aufgeklappten Zustand;
**Fig. 4****:** eine erfindungsgemäße Wärmeübertragungseinheit gemäß **Fig.** 1 in Kombination mit einer erfindungsgemäßen Druckeinrichtung gemäß **Fig.** 3; und
**Fig. 5****:** ein Flussdiagramm eines erfindungsgemäßen Verfahrens zum Betreiben der Vorrichtung.

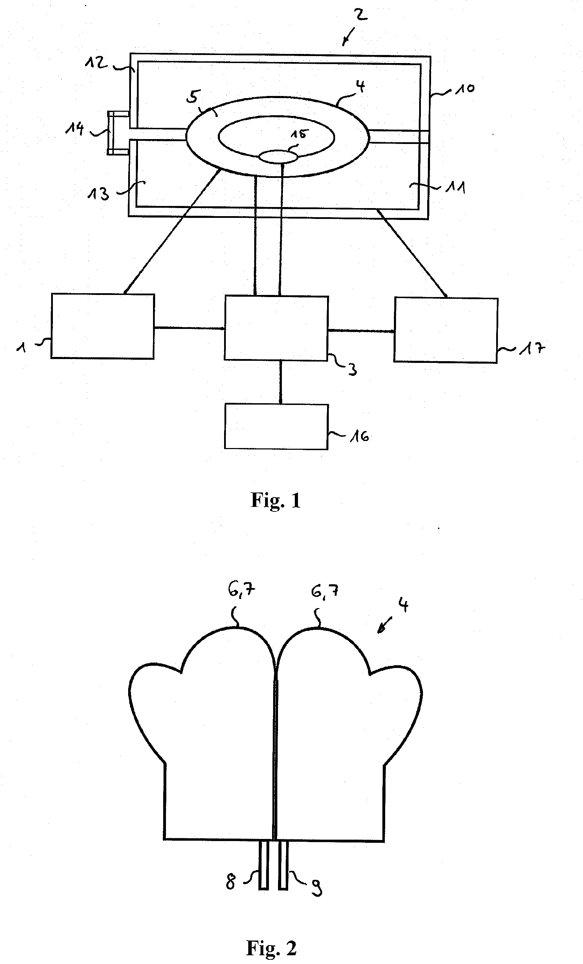

**Fig.** 1 zeigt eine Vorrichtung zur Verwendung bei der wärmetherapeutischen Behandlung eines ausgewählten Bereiches des Körpers eines Menschen oder eines Tieres, und insbesondere zur Verwendung bei der Behandlung von Gewebeschädigungen nach operativen Eingriffen, von Verletzungen, von Entzündungen und von chronischen Erkrankungen des rheumatischen Formenkreises, und insbesondere zur Unterstützung von chemotherapeutischen Behandlungen, mit einer Temperiereinrichtung 1, die dazu vorgesehen ist, ein Temperierfluid zu temperieren. Die Temperiereinrichtung 1 ist mit einer Wärmeübertragungseinrichtung 2, die zur Übertragung von Wärme auf den ausgewählten Bereich des Körpers eingerichtet ist, fluidisch verbunden, und ferner eingerichtet ist, die Wärmeübertragungseinrichtung 2, mit einem, die Wärmeübertragungseinrichtung 2, durchströmenden, wärmeübertragenden Temperierfluid, zu temperieren. Weiterhin ist eine Steuereinrichtung 3 mit der Temperiereinrichtung 1 und der Wärmeübertragungseinrichtung 2 verbunden, wobei die Steuereinrichtung 3 eingerichtet ist, die Temperiereinrichtung 1 anzusteuern, um zu bewirken, dass die Wärmeübertragungseinrichtung 2, im Bereich einer Kontaktfläche zwischen der Wärmeübertragungseinrichtung 2, und dem zu temperierenden ausgewählten Bereich des Körpers zumindest teilweise eine in etwa gleichmäßige Temperatur aufweist.

Die Wärmeübertragungseinrichtung 2 weist dabei wenigstens eine Wärmeübertragungseinheit 4 auf, welche dafür vorgesehen ist, in Kontakt mit dem ausgewählten Bereich des Körpers gebracht zu werden, wobei die Wärmeübertragungseinheit 4 aus einem, im wesentlichen flexiblen Material besteht, in welchem eine oder mehrere Temperierfluidkammern 5 angeordnet sind, die von dem Temperierfluid durchströmbar sind.

Die Wärmübertragungseinrichtung weist dabei ferner eine, an die Wärmeübertragungseinheit 4 angrenzende, Druckeinrichtung 10 auf, wobei die Druckeinrichtung 10 wenigstens eine Druckeinheit 11 und eine Abstützeinrichtung 12 aufweist.

Die Druckeinheit 11 steht mit der Abstützeinrichtung 12 in Verbindung und die Druckeinheit 11 ist dazu vorgesehen, die Wärmeübertragungseinheit 4 wenigstens teilweise mit einem Druck zu beaufschlagen, welcher dann als Anliegedruck der Wärmeübertragungseinrichtung 2 vom zu behandelnden Teil des Körpers aufgenommen wird. Die Druckeinheit 11 weist hierfür wenigstens eine Druckkammer 13, mit einem vorbestimmten oder einen veränderbaren Innendruck, auf, und ist dafür eingerichtet, einen Anliegedruck der Wärmeübertragungseinrichtung 2 auf den ausgewählten Bereich wenigstens teilweise zu verstärken. Die wenigstens eine Druckkammer 13 umfasst dabei eine mit einem Druckfluid gefüllte Kammer, wobei die wenigstens eine mit einem Druckfluid gefüllte Kammer durch ein flexibles Material gebildet ist. Das Druckfluid in der wenigstens einen mit einem Druckfluid gefüllten Druckkammer kann dabei, über eine Druckleitung, die mit der wenigstens einen mit einem Druckfluid gefüllten Kammer verbunden ist, entweder manuell über Muskelkraft und/oder unter Einsatz eines Verdichters 17, mit Druck beaufschlagt werden, um einen Innendruck in der wenigstens einen mit einem Druckfluid gefüllten Kammer zu verändern.

Die Abstützeinrichtung 12 der Druckeinrichtung 10 weist ferner eine Verbindungseinrichtung 14 mit wenigstens einem Fixierelement auf, und die Verbindungseinrichtung 14 ist derart ausgebildet, dass sie die Druckeinrichtung 10 zumindest teilweise umgreift und in dieser umgreifenden Stellung Stützkräfte auf die Druckeinrichtung 10 ausübt, welche zumindest teilweise dazu beitragen, die Druckeinrichtung 10 in einem Benutzungszustand an dem ausgewählten Bereich des Körpers eines Menschen oder eines Tieres zu fixieren.

Ferner ist wenigstens eine Sensoreinrichtung 15 vorgesehen, die mit der Steuereinrichtung 3 verbunden ist. Die Sensoreinrichtung 15 ist eingerichtet zwischen dem ausgewählten Bereich des Körpers und der Hautkontaktfläche ein Sensorsignal zu messen. Das Sensorsignal kann dabei optisch, akustisch oder digital vorliegen. In dem in Fig. 1 gezeigten Ausführungsbeispiel ist die Sensoreinrichtung 15 zwischen der Wärmeübertragungseinheit 4 und dem zu behandelnden Teil des Körpers angebracht, es sind jedoch auch andere Positionen möglich.

Ferner ist wenigstens eine Anzeigeeinrichtung 16 vorgesehen, wobei die Anzeigeeinrichtung 16 mit der Steuereinrichtung 3 verbunden und eingerichtet ist, das von der Sensoreinrichtung 15 gemessene Sensorsignal über die Anzeigeeinrichtung 16 an einen Benutzer auszugeben. Das Sensorsignal kann auch für eine Regelung genutzt werden, so dass ein Bereich einer Kontaktfläche zwischen der Wärmeübertragungseinrichtung 2, und dem zu temperierenden Teils des Körpers zumindest in einem Bereich eine in etwa gleichmäßige, vorgewählte Temperatur aufweist.

**Fig.** 2 zeigt eine Wärmeübertragungseinheit 4, welche dafür vorgesehen ist, in Kontakt mit dem ausgewählten Bereich des Körpers gebracht zu werden, wobei die Wärmeübertragungseinheit 4 aus einem im wesentlichen flexiblen Material besteht, in welchem eine oder mehrere Temperierfluidkammern 5 (nicht gezeigt) angeordnet sind, die von dem Temperierfluid durchströmbar sind.

Die Wärmeübertragungseinheit 4 weist dabei auf:
- wenigstens eine erste flexible Materiallage 6 mit wenigstens einer ersten Hautkontaktfläche, welche zumindest teilweise einem ausgewählten Bereich des Körpers eines Menschen oder eines Tieres zugewandt ist, und mit wenigstens einer von der ersten Hautkontaktfläche abgewandten ersten Temperierfluidkontaktfläche,
- wenigstens eine zweite flexible Materiallage 7 mit wenigstens einer zweiten Temperierfluidkontaktfläche, die zwischen sich und der ersten Temperierfluidkontaktfläche der ersten flexiblen Materiallage 6 zumindest teilweise einen oder mehrere von einem wärmeübertragenden Temperierfluid durchströmbare Temperierfluidkammer begrenzt,
wobei die Wärmeübertragungseinheit 4 weiterhin aufweist:
- wenigstens eine erste Zuleitung 8, um der Wärmeübertragungseinheit 4 ein wärmeübertragendes Temperierfluid zuzuführen,
- wenigstens eine zweite Zuleitung 9, um ein wärmeübertragendes Temperierfluid von der Wärmeübertragungseinheit 4 abzuführen.

Die Verwendung von flexiblen Materiallagen 6, 7 ist notwendig, da der zu temperierende Bereich des Körpers nicht eben ist, sondern konkave und/oder konvexe Bereiche aufweist, und die Flexibilität der Materiallagen 6, 7 es ermöglicht den Abstand zwischen Wärmeübertragungseinheit 4 und dem zu temperierenden Bereich über die ganze dreidimensionale Fläche möglichst gering zu halten, wobei die Wärmeübertragungseinheit 4 bevorzugt unmittelbar auf der Hautoberfläche aufliegt.

Die Wärmeübertragungseinheit 4 weist dabei wenigstens eine erste Form auf oder kann eine solche erste Form annehmen, in welcher sie den ausgewählten Bereich des Körpers eines Menschen oder eines Tieres zumindest teilweise umschließt.

In einer bevorzugten Variante weist die Wärmeübertragungseinheit 4 eine der folgenden Formen auf, oder kann eine dieser Formen annehmen:
- die Form einer die Hand umschließende Hülle ohne Unterteilungen des Innenraums;
- die Form eines Handschuhs mit teilweise abgeteilten Umhüllungen für wenigstens einen Finger, insbesondere die Form eines Fausthandschuhs mit einer teilweise abgeteilten Umhüllung für den Daumen, wie in **Fig.** 2 gezeigt;
- die Form eines Fingerhandschuhs mit teilweise abgeteilten Umhüllungen für jeden Finger;
- die Form eines zumindest teilweise fingerlosen Handschuhs, bei dem wenigstens ein Finger nicht umschlossen ist.

Der zu behandelnde Körperteil kann jedoch auch ein Fuß oder ein anderer Körperteil sein, wobei die Wärmeübertragungseinheit **2** dann eine Schuh- oder Strumpfform bzw. eine andere, an den jeweiligen Körperteil angepasste Form aufweist oder annehmen kann.

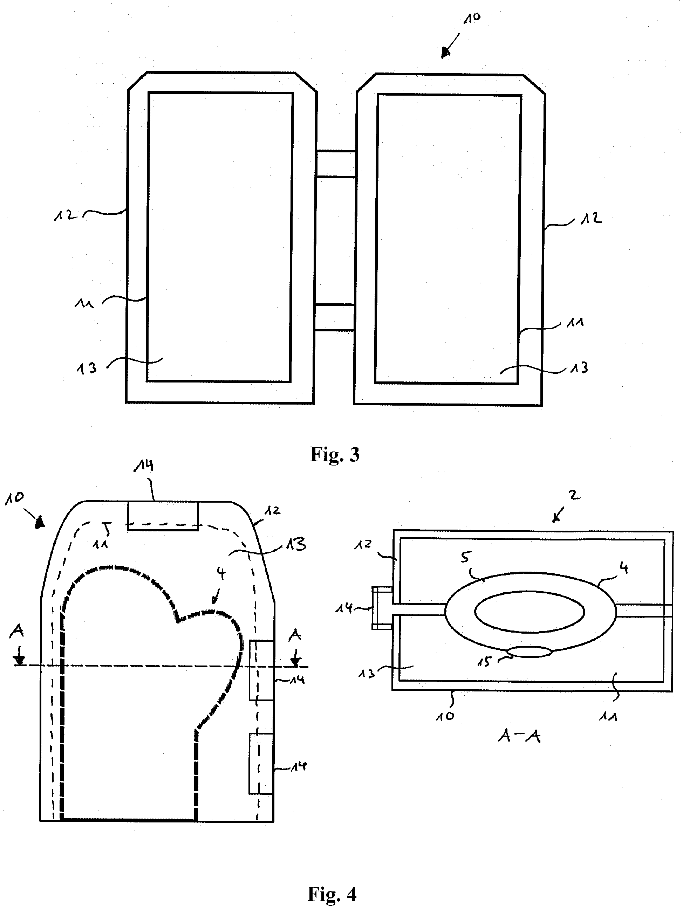

**Fig.** 3 zeigt die Druckeinrichtung 10, wobei die Druckeinrichtung 10 wenigstens eine Druckeinheit 11 und eine Abstützeinrichtung 12, die dafür vorgesehen ist einen Anliegedruck aufzubauen, aufweist. Die Druckeinheit 11 ist an der Abstützeinrichtung 12 angebracht und dazu vorgesehen, die Wärmeübertragungseinheit 4 wenigstens teilweise mit einem Druck zu beaufschlagen. Hierfür weist die Druckeinheit 11 wenigstens eine Druckkammer 13 auf, die einen vorbestimmten oder einen veränderbaren Innendruck aufweist, und dafür eingerichtet ist, den Anliegedruck der Wärmeübertragungseinrichtung 2 auf den ausgewählten Bereich über den Innendruck wenigstens teilweise zu verstärken. Die Druckeinheit 11 kann dabei gemäß einer Ausführungsform eine Druckkammer 13 in Form einer mit einem Druckfluid gefüllten Kammer aufweisen, wobei die wenigstens eine mit einem Druckfluid gefüllte Kammer durch ein flexibles Material umgrenzt ist. In einer weiteren bevorzugten Ausführungsform ist die wenigstens eine Druckeinheit 11 aus einem elastischen Material hergestellt, wie vorzugsweise einem Schaumstoff, einem Gummi, oder einer Kombination dieser oder ähnlicher Materialien, wobei die Druckeinheit 11 und die Druckkammer 13 in dieser Ausführungsform als dasselbe Bauteil anzusehen sind.

**Fig**. 4 zeigt erneut die Wärmeübertragungseinrichtung 2 aus **Fig.** 1 und die Druckeinrichtung 10 aus **Fig**. 3 in leicht abgewandter Dimensionierung. Hierbei stellt die rechte Figur einen Schnitt A-A durch die Schnittebene aus der Zeichenebene heraus entlang der strichlierten Gerade A-A in der linken Figur dar. In der linken Figur ist die Wärmeübertragungseinheit 4 aus **Fig**. 2 ebenfalls strichliert angedeutet. Die Wärmeübertragungseinrichtung 2 umfasst hierbei wenigstens eine Wärmeübertragungseinheit 4, in welchem eine oder mehrere Temperierfluidkammern 5 angeordnet sind. An der Wärmeübertragungseinheit 4 liegt eine Druckeinrichtung 10 an, wobei die Druckeinrichtung 10 wenigstens eine Druckeinheit 11, und eine Abstützeinrichtung 12 aufweist. Die Abstützeinrichtung 12 ist an der Druckeinheit 11 angebracht und dazu vorgesehen, über die Wärmeübertragungseinheit 4 wenigstens teilweise einen Anliegedruck der Wärmeübertragungseinrichtung 2 auf den zu behandelnden Teil des Körpers aufzubauen. Hierfür weist die Druckeinheit 11 wenigstens eine Druckkammer 13 auf. Die Druckkammer 13 ist dafür eingerichtet, über einen Innendruck den Anliegedruck der Wärmeübertragungseinrichtung 2 auf den ausgewählten Bereich wenigstens teilweise zu verstärken. Die Druckeinrichtung 10 weist ferner eine Abstützeinrichtung 12 auf, wobei die Druckeinrichtung 10 an der Abstützeinrichtung 12 angebracht ist, und die Druckeinheit 11 dazu vorgesehen ist, die Wärmeübertragungseinheit 4 wenigstens teilweise mit einem Druck zu beaufschlagen. Die Druckeinrichtung weist in dieser Ausführungsform drei Verbindungseinrichtungen 14 auf, vorzugsweise sind allerdings auch mehr oder weniger Verbindungseinrichtungen denkbar. Die gezeigten Verbindungseinrichtungen 14 sind in dieser Ausführungsform jeweils einteilig ausgeführt, in einer bevorzugten Ausführungsform ist die Verbindungseinrichtung umlaufend gestaltet, beispielsweise in Form eines Reisverschlusses oder eines ähnlichen Mechanismus. In der in **Fig.** 4 gezeigten Ausführungsform ist die Druckeinheit 11 in der Draufsicht großflächiger als die Wärmeübertragungseinheit 4, sowie einteilig ausgestaltet, vorzugsweise sind jedoch auch im Verhältnis zur Wärmeübertragungseinheit kleinere Dimensionierungen und mehrteilige Druckeinheiten denkbar, welche einzeln oder gemeinsam, durch eine gemeinsame Fluidverbindung, gefüllt, oder, unter Einsatz einer Steuereinrichtung 3 angesteuert werden können.

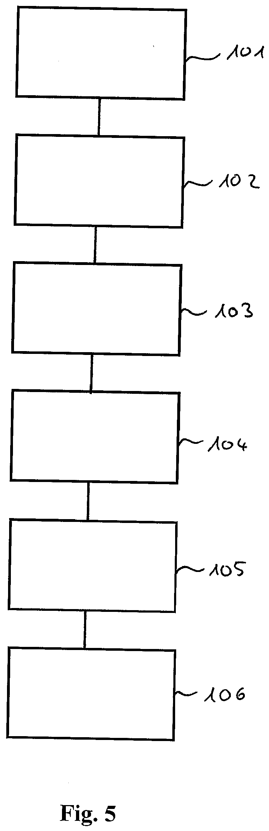

**Fig.** 5 zeigt ein Flussdiagramm eines Verfahrens zum Betreiben einer Vorrichtung zur wärmetherapeutischen Behandlung eines ausgewählten Bereiches des Körpers eines Menschen oder eines Tieres gemäß der Erfindung, wobei das Verfahren folgende Schritte aufweist:
- In Kontakt bringen 101 einer Wärmeübertragungseinrichtung 2 mit einer Druckeinrichtung 10 an den ausgewählten Bereich des Körpers in eine Benutzungszustand;
- Anlegen 102 eines auf den ausgewählten Bereich des Körpers wirkenden Anliegedrucks über die Druckeinrichtung 10,
- Temperieren 103, über eine mit der Wärmeübertragungseinrichtung 2 verbundene Temperiereinrichtung 1 des ausgewählten Bereiches des Körpers indem eine Wärmeübertragungseinheit 4 der Wärmeübertragungseinrichtung 2 von einem wärmeübertragenden Temperierfluid durchströmt wird;
sodass im Bereich einer Kontaktfläche zwischen der Wärmeübertragungseinrichtung 2, und dem zu temperierenden Teils des Körpers zumindest in einem Bereich eine in etwa gleichmäßige Temperatur aufweist.

In einer bevorzugten Ausführungsform weist das Verfahren zum Betreiben einer Vorrichtung zur wärmetherapeutischen Behandlung ferner folgenden Schritt auf:
- Einstellen 104 eines Innendrucks einer Druckeinheit 11 der Druckeinrichtung 10, wobei die Druckeinheit 11 wenigstens eine mit einem Druckfluid gefüllte Kammer aufweist, wobei der Innendruck der wenigstens einen mit einem Druckfluid gefüllten Kammer manuell über Muskelkraft eines Benutzers und/oder über eine externe Druckquelle einstellbar ist.

In einer weiter bevorzugten Ausführungsform weist das Verfahren zum Betreiben einer Vorrichtung zur wärmetherapeutischen Behandlung ferner folgende Schritte auf:
- Messen 105 eines Sensorsignals, über eine mit der Wärmeübertragungseinrichtung 2 verbundene Sensoreinrichtung 15;
- Steuern 106, über eine Steuereinrichtung 3, der mit der Steuereinrichtung 3 verbundenen Druckeinrichtung 10, basierend auf dem gemessenen Sensorsignal.

Die Steuereinrichtung 3 ist dabei vorzugsweise derart eingerichtet, dass sie mit der Wärmeübertragungseinrichtung 2 und der Temperiereinrichtung 1 unter Verwendung des von der Sensoreinrichtung 15 gemessenen Sensorsignals einen Regelkreis bildet, so dass im Bereich einer Kontaktfläche zwischen der Wärmeübertragungseinrichtung, und dem zu temperierenden Teils des Körpers zumindest in einem Bereich eine in etwa gleichmäßige, vorgewählte Temperatur geregelt wird. Die Wärmeübertragung ist dabei durch das Vermeiden von thermisch isolierenden Luftpolstern zwischen Wärmeübertragungseinheit und zu temperierendem Bereich des Körpers deutlich verbessert.

### Bezugszeichenliste

- **1**: Temperiereinrichtung
- **2**: Wärmeübertragungseinrichtung
- **3**: Steuereinrichtung
- **4**: Wärmeübertragungseinheit
- **5**: Temperierfluidkammer
- **6**: erste flexible Materiallage
- **7**: zweite flexible Materiallage
- **8**: erste Zuleitung
- **9**: zweite Zuleitung
- **10**: Druckeinrichtung
- **11**: Druckeinheit
- **12**: Abstützeinrichtung
- **13**: Druckkammer
- **14**: Verbindungseinrichtung
- **15**: Sensoreinrichtung
- **16**: Anzeigeeinrichtung
- **17**: Verdichter
- **101**: In Kontakt bringen
- **102**: Anlegen
- **103**: Temperieren
- **104**: Einstellen
- **105**: Messen
- **106**: Steuern

## Patentansprüche

1. Vorrichtung zur Verwendung bei der wärmetherapeutischen Behandlung eines ausgewählten Bereiches des Körpers eines Menschen oder eines Tieres, und insbesondere zur Verwendung bei der Behandlung von Gewebeschädigungen nach operativen Eingriffen, von Verletzungen, von Entzündungen und von chronischen Erkrankungen des rheumatischen Formenkreises, und insbesondere zur Unterstützung von chemotherapeutischen Behandlungen, mit:
einer Temperiereinrichtung (**1**), die dazu vorgesehen ist, ein Temperierfluid zu temperieren;
einer Wärmeübertragungseinrichtung (**2**), die zur Übertragung von Wärme auf den ausgewählten Bereich des Körpers eingerichtet ist,
wobei die Temperiereinrichtung (**1**) mit der Wärmeübertragungseinrichtung (**2**), fluidisch verbunden ist, und ferner eingerichtet ist, die Wärmeübertragungseinrichtung (**2**), mit einem, die Wärmeübertragungseinrichtung (**2**), durchströmenden, wärmeübertragenden Temperierfluid, zu temperieren,
eine Steuereinrichtung (**3**), die mit der Temperiereinrichtung (**1**) und der Wärmeübertragungseinrichtung (**2**), verbunden ist,
wobei die Steuereinrichtung (**3**) eingerichtet ist, die Temperiereinrichtung (**1**) anzusteuern, und um zu bewirken,
dass die Wärmeübertragungseinrichtung (**2**), im Bereich einer Kontaktfläche zwischen der Wärmeübertragungseinrichtung (**2**), und dem zu temperierenden ausgewählten Bereich des Körpers zumindest teilweise eine in etwa gleichmäßige Temperatur aufweist.

2. Vorrichtung gemäß Anspruch 1, **dadurch gekennzeichnet, dass** die Wärmeübertragungseinrichtung (**2**) wenigstens eine Wärmeübertragungseinheit (**4**) aufweist, welche dafür vorgesehen ist, in Kontakt mit dem ausgewählten Bereich gebracht zu werden, wobei die Wärmeübertragungseinheit (**4**) aus einem im wesentlichen flexiblen Material besteht, in welchem eine oder mehrere Temperierfluidkammern (**5**) angeordnet sind, die von dem Temperierfluid durchströmbar sind.

3. Vorrichtung gemäß Anspruch 2, **dadurch gekennzeichnet, dass** die Wärmeübertragungseinheit (**4**) aufweist:
wenigstens eine erste flexible Materiallage (**6**) mit wenigstens einer ersten Hautkontaktfläche, welche zumindest teilweise einem ausgewählten Bereich des Körpers eines Menschen oder eines Tieres zugewandt ist, und mit wenigstens einer von der ersten Hautkontaktfläche abgewandten ersten Temperierfluidkontaktfläche,
wenigstens eine zweite flexible Materiallage (**7**) mit wenigstens einer zweiten Temperierfluidkontaktfläche, die zwischen sich und der ersten Temperierfluidkontaktfläche der ersten flexiblen Materiallage (**6**) zumindest teilweise einen oder mehrere von einem wärmeübertragenden Temperierfluid durchströmbare Temperierfluidkammer begrenzt,
wobei die Wärmeübertragungseinheit (**4**) weiterhin aufweist:
wenigstens eine erste Zuleitung (**8**), um der Wärmeübertragungseinheit (**4**) ein wärmeübertragendes Temperierfluid zuzuführen,
wenigstens eine zweite Zuleitung (**8**), um ein wärmeübertragendes Temperierfluid von der Wärmeübertragungseinheit (**4**) abzuführen, wobei die Wärmeübertragungseinheit (**4**) wenigstens eine erste Form aufweist oder eine solche erste Form annehmen kann, in welcher sie den ausgewählten Bereich des Körpers eines Menschen oder eines Tieres zumindest teilweise umschließt.

4. Vorrichtung gemäß mindestens einem der vorhergehenden Ansprüche, **dadurch gekennzeichnet, dass** die Wärmübertragungseinrichtung ferner eine Druckeinrichtung (**10**) aufweist,
wobei die Druckeinrichtung (**10**) aufweist:
wenigstens eine Druckeinheit (**11**), und
eine Abstützeinrichtung (**12**),
wobei die Druckeinrichtung (**10**) an der Abstützeinrichtung (**12**) angebracht ist und die Druckeinheit (**11**) dazu vorgesehen ist, die Wärmeübertragungseinheit (**4**) wenigstens teilweise mit einem Druck zu beaufschlagen, wobei die Druckeinheit (**11**) wenigstens eine Druckkammer (**13**) aufweist, die einen vorbestimmten oder einen veränderbaren Innendruck aufweist, und dafür eingerichtet ist, einen Anliegedruck der Wärmeübertragungseinrichtung (**2**) auf den ausgewählten Bereich wenigstens teilweise zu verstärken.

5. Vorrichtung gemäß Anspruch 4, **dadurch gekennzeichnet, dass** die wenigstens eine Druckkammer (**13**) eine mit einem Druckfluid gefüllte Kammer umfasst, wobei die wenigstens eine mit einem Druckfluid gefüllte Kammer durch ein flexibles Material gebildet ist.

6. Vorrichtung gemäß Anspruch 5, **dadurch gekennzeichnet, dass** die wenigstens eine mit einem Druckfluid gefüllte Kammer eine Mehrzahl von mit einem Druckfluid gefüllten Kammern ist.

7. Vorrichtung gemäß mindestens einem der Ansprüche 5 oder 6, **dadurch gekennzeichnet, dass** das Druckfluid ausgewählt ist aus der Gruppe von Luft, Wasser, Öl oder eine Kombination hiervon.

8. Vorrichtung gemäß mindestens einem Ansprüche 5 bis 7, **dadurch gekennzeichnet, dass** das Druckfluid in der wenigstens einen mit einem Druckfluid gefüllten Kammer entweder:
manuell über Muskelkraft,
und/oder
über einen Verdichter (**17**)
über eine Druckleitung, die mit der wenigstens einen mit einem Druckfluid gefüllten Kammer verbunden ist, mit Druck beaufschlagbar ist, um einen Innendruck in der wenigstens einen mit einem Druckfluid gefüllten Kammer zu verändern.

9. Vorrichtung gemäß Anspruch 4, **dadurch gekennzeichnet, dass** die wenigstens eine Druckkammer (**13**) aus einem elastischen Material hergestellt ist und zwischen der Abstützeinrichtung (**12**) und der Wärmeübertragungseinheit (**4**) angeordnet ist.

10. Vorrichtung gemäß mindestens einem der Ansprüche 4 bis 9, **dadurch gekennzeichnet, dass** die Abstützeinrichtung (**12**) der Druckeinrichtung (**10**) aus einem wenig flexiblen Material hergestellt ist.

11. Vorrichtung gemäß mindestens einem der Ansprüche 4 bis 10, **dadurch gekennzeichnet, dass** die Abstützeinrichtung (**12**) der Druckeinrichtung (**10**) um den ausgewählten Bereich des Körpers eines Menschen oder Tieres ferner eine Verbindungseinrichtung (**14**) mit wenigstens einem Fixierelement aufweist, und die Verbindungseinrichtung (**14**) derart ausgebildet ist, dass sie die Druckeinrichtung (**10**) zumindest teilweise umgreift, und in dieser umgreifenden Stellung Stützkräfte auf die Druckeinrichtung (**10**) ausübt, welche zumindest teilweise dazu beitragen, die Druckeinrichtung (**10**) in einem Benutzungszustand an dem ausgewählten Bereich des Körpers eines Menschen oder eines Tieres zu fixieren.

12. Vorrichtung gemäß mindestens einem der vorhergehenden Ansprüche, **dadurch gekennzeichnet, dass** wenigstens eine Sensoreinrichtung (**15**) vorgesehen ist, die mit der Steuereinrichtung (**3**) verbunden ist,
wobei die Sensoreinrichtung (**15**) eingerichtet ist zwischen dem ausgewählten Bereich des Körpers und der Hautkontaktfläche ein Sensorsignal zu messen.

13. Vorrichtung gemäß Anspruch 12, **dadurch gekennzeichnet, dass** die wenigstens eine Sensoreinrichtung (**15**) eine Mehrzahl von Sensoreinrichtungen ist, die zumindest teilweise in einem Raster auf der Hautkontaktfläche der ersten Materiallage angebracht sind.

14. Vorrichtung gemäß mindestens einem der Ansprüche 12 oder 13, **dadurch gekennzeichnet, dass** das gemessene Sensorsignal ausgewählt ist aus der Gruppe Druck, Stromstärke, Temperatur.

15. Vorrichtung gemäß mindestens einem der vorhergehenden Ansprüche, **dadurch gekennzeichnet, dass** wenigstens eine Anzeigeeinrichtung (**16**) vorgesehen ist,
wobei die Anzeigeeinrichtung (**16**) mit der Steuereinrichtung (**3**) verbunden und eingerichtet ist, das von der Sensoreinrichtung (**15**) gemessene Sensorsignal über die Anzeigeeinrichtung (**16**) an einen Benutzer auszugeben.

16. Vorrichtung gemäß mindestens einem der vorhergehenden Ansprüche, **dadurch gekennzeichnet, dass** die Temperiereinrichtung (**1**) aufweist:
wenigstens ein Gehäuse,
wenigstens eine Temperierfluidversorgungseinrichtung, durch welche ein wärmeübertragendes Temperierfluid in dem Gehäuse bereitgestellt wird,
wenigstens eine im Gehäuse angeordneten Temperiereinheit, die das durch das Gehäuse strömende wärmeübertragende Temperierfluid temperiert,
wenigstens eine Temperierfluidabströmungseinrichtung, durch welche das wärmeübertragende Temperierfluid mit einer im Wesentlichen vorbestimmten Temperatur aus dem Gehäuse herausgeführt wird,
wenigstens eine im Gehäuse angeordneten Pumpeneinrichtung, welche das durch das Gehäuse strömende wärmeübertragende Temperierfluid mit Druck beaufschlagt,
wobei diese wenigstens eine Temperiereinheit wenigstens ein erstes Temperierelement und wenigstens ein zweites Temperierelement aufweist, welche jeweils wenigstens ein thermoelektrisches Element, insbesondere wenigstens ein Peltier-Element, wenigstens ein von einem Temperierfluid durchströmbares Wärmetauscherelement zur Übertragung von Wärme auf das oder von dem Temperierfluid und wenigstens einen Kühlkörper aufweisen.

17. Vorrichtung gemäß mindestens einem der Ansprüche 12 bis 16, **dadurch gekennzeichnet, dass** die Steuereinrichtung (**3**) eingerichtet ist, über das von der Sensoreinrichtung (**15**) gemessene Sensorsignal und der Temperiereinrichtung (**1**) einen Regelkreis derart zu bilden,
dass ein Bereich einer Kontaktfläche zwischen der Wärmeübertragungseinrichtung (**2**), und dem zu temperierenden Teils des Körpers zumindest in einem Bereich eine in etwa gleichmäßige Temperatur aufweist

18. Vorrichtung gemäß mindestens einem der vorhergehenden Ansprüche, **dadurch gekennzeichnet, dass** die Vorrichtung zum Kühlen eine Temperierung des Temperierfluids in einem Temperaturbereich von 10°C bis 35 °C, vorzugsweise von 12°C bis 22 °C, weiter vorzugsweise von 15°C bis 22 °C, weiter vorzugsweise 10°C bis 35 °C, oder aber ebenfalls vorzugsweise von 10°C bis 43 °C ermöglicht.

19. Vorrichtung gemäß mindestens einem der Ansprüche 1 bis 17, **dadurch gekennzeichnet, dass** die Vorrichtung zum Wärmen eine Temperierung des Temperierfluids in einem Temperaturbereich von 35°C bis 45 °C, vorzugsweise von 38°C bis 44 °C, weiter vorzugsweise von 41 °C bis 43 °C ermöglicht.

20. Verfahren zum Betreiben einer Vorrichtung zur wärmetherapeutischen Behandlung eines ausgewählten Bereiches des Körpers eines Menschen oder eines Tieres, insbesondere mit einer Vorrichtung nach einem der Ansprüche 4 bis 19, wobei das Verfahren folgende Schritte aufweist:
- In Kontakt bringen (**101**) einer Wärmeübertragungseinrichtung (**2**) mit einer Druckeinrichtung (**10**) an den ausgewählten Bereich des Körpers in einen Benutzungszustand;
- Anlegen (**102**) eines auf den ausgewählten Bereich des Körpers wirkenden Anliegedrucks über die Druckeinrichtung (**10**),
- Temperieren (**103**), über eine mit der Wärmeübertragungseinrichtung (**2**) verbundene Temperiereinrichtung (**1**) des ausgewählten Bereiches des Körpers indem eine Wärmeübertragungseinheit (**4**) der Wärmeübertragungseinrichtung (**2**) von einem wärmeübertragenden Temperierfluid durchströmt wird; sodass im Bereich einer Kontaktfläche zwischen der Wärmeübertragungseinrichtung (**2**), und dem zu temperierenden Teils des Körpers zumindest in einem Bereich eine in etwa gleichmäßige Temperatur aufweist.

21. Verfahren gemäß Anspruch 20, **dadurch gekennzeichnet, dass** das Verfahren ferner folgenden Schritt aufweist:
- Einstellen (**104**) eines Innendrucks einer Druckeinheit (**11**) der Druckeinrichtung (**10**), wobei die Druckeinheit (**11**) wenigstens eine mit einem Druckfluid gefüllte Druckkammer (**13**) aufweist, wobei der Innendruck der wenigstens einen mit einem Druckfluid gefüllten Druckkammer (**13**) manuell über Muskelkraft eines Benutzers und/oder über eine externe Druckquelle einstellbar ist.

22. Verfahren gemäß mindestens einem der Ansprüche 20 oder 21, **dadurch gekennzeichnet, dass** das Verfahren ferner folgende Schritte aufweist:
- Messen (**105**) eines Sensorsignals, über eine mit der Wärmeübertragungseinrichtung (**2**) verbundene Sensoreinrichtung (**15**);
- Steuern (**106**), über eine Steuereinrichtung (**3**), der mit der Steuereinrichtung (**3**) verbundenen Druckeinrichtung (**10**), basierend auf dem gemessenen Sensorsignal.
